Europäisches Patentamt

European Patent Office

Office européen des brevets

(19)

(11) Veröffentlichungsnummer: **0 039 494**
**A1**

# EUROPÄISCHE PATENTANMELDUNG

(12)

(21) Anmeldenummer: 81103293.7

(22) Anmeldetag: 01.05.81

(51) Int. Cl.³: **G 01 M 19/00**

(30) Priorität: 06.05.80 DE 3017206
06.05.80 DE 3017242

(71) Anmelder: Till, Heinz, Fischbacher Weg 28,
D-6238 Hofheim (DE)
Anmelder: Till, Volker, Eichendorffstrasse 17,
D-6238 Hofheim (DE)

(43) Veröffentlichungstag der Anmeldung: 11.11.81
Patentblatt 81/45

(72) Erfinder: Till, Heinz, Fischbacher Weg 28,
D-6238 Hofheim (DE)
Erfinder: Till, Volker, Eichendorffstrasse 17,
D-6238 Hofheim (DE)

(84) Benannte Vertragsstaaten: AT BE CH FR GB IT LI LU NL
SE

(74) Vertreter: Keil, Rainer, Dipl.-Phys. Dr.,
Ammelburgstrasse 34, D-6000 Frankfurt am Main 1 (DE)

(54) Verfahren und Vorrichtung zur Überprüfung und Gewährleistung der Brauchbarkeit von Kunststoffbehältern mit Griffleisten, z.B. Flaschentransportkästen.

(57) Die Erfindung bezieht sich auf ein Verfahren und eine Vorrichtung zur Überprüfung und Gewährleistung der Brauchbarkeit von Kunststoffbehältern mit Griffleisten, z.B. Flaschentransportkästen aus Polyäthylen oder dgl. Ein einfaches und zuverlässiges Aussortieren von Kunststoffkästen mit gebrochener oder gerissener Griffleiste zeichnet sich dadurch aus, dass man die Kunststoffbehälter (3) innerhalb der Produktionslinie im Bereich der Griffleiste (4) auf Brüche oder Risse selbsttätig mechanisch oder mit Hilfe eines Ultraschall-Prüfverfahrens, z.B. des Ultraschall-Durchschallung- und/oder Impulsecho-Verfahrens abtastet und bei Feststellung eines Bruches oder Risses aus der Produktionslinie ausstösst.

0039494
MERTENS & KEIL
PATENTANWÄLTE

Heinz Till

Fischbacher Weg 28

6238 Hofheim


Volker Till

Eichendorffstr. 17

6238 Hofheim


"Verfahren und Vorrichtung zur Überprüfung und Gewährleistung
der Brauchbarkeit von Kunststoffbehältern mit Griffleisten,
z.B. Flaschentransportkästen"

Die Erfindung betrifft ein Verfahren zur Überprüfung und Gewährleistung der Brauchbarkeit von Kunststoffbehältern mit Griffleisten, z.B. Flaschentransportkästen aus Polyäthylen oder
dergl. sowie eine Vorrichtung zur Durchführung des Verfahrens.

In Brauereien und anderen Getränkebetrieben, die Kunststoffkästen mit Griffleisten, z.B. Flaschentransportkästen in Benutzung haben, kommt es insbesondere auch alterungsbedingt
häufig zum Bruch der Griffleisten. Ein solcher Bruch führt
innerhalb der Produktionslinie zu erheblichen Störungen, z.B.
bei den Palettiermaschinen, in welchen der Kunststoffkasten
an der Griffleiste erfaßt und transportiert werden muß, insbesondere bei Beladung mit Vollgut. Außerdem sind Kunststoffbehälter mit gebrochenen Griffleisten eine Gefahr für die
Standfestigkeit bei der Stapelung.

Aufgabe der vorliegenden Erfindung ist es, ein Verfahren und
eine Vorrichtung zur einfachen und zuverlässigen Aussortierung
von Kunststoffkästen mit gebrochenen oder gerissenen Griffleisten vorzuschlagen.

0039494

MERTENS & KEIL
PATENTANWÄLTE

Eine manuelle Aussortierung würde einen erheblichen personellen Aufwand bedeuten, wenn man bedenkt, daß bereits in Brauereien mittlerer Größe Abfüllanlagen installiert sind, die stündlich Tausende von Kunststoffkästen umschlagen. Zudem ist der teilweise oder bereits eingetretene Bruch einer Griffleiste mit bloßem Auge häufig nicht leicht erkennbar.

Gemäß der Erfindung wird zur Lösung des genannten Problems vorgeschlagen, daß man die Kunststoffbehälter innerhalb einer Produktionslinie im Bereich der Griffleisten auf Brüche oder Risse selbständig mechanisch oder mit Hilfe eines Ultraschall-Prüfungsverfahrens, z.B. des Ultraschall-Durchschallung- und/oder Impulsecho-Verfahrens abtastet und bei Feststellung eines Bruches oder Risses aus der Produktionslinie ausstößt.

Auf diese Weise werden alle, aber auch nur diejenigen Kunststoffbehälter ausgeschieden, bei welchen tatsächlich bereits ein Bruch der Griffleiste eingetreten ist. Dabei macht sich die Erfindung den Umstand zunutze, daß die Benutzungsbeanspruchung der Kunststoffbehälter außerhalb der Brauerei oder des Getränkebetriebes in der Regel höher ist als innerhalb der Produktionslinie, d.h. der Bruch einer Griffleiste mit überwiegender Wahrscheinlichkeit außerhalb der Brauerei oder des Getränkebetriebes auftritt. Die Überprüfung der Brauchbarkeit durch Abtastung bereits gebrochener oder durch Riß beschädigter Griffleisten kann sich also auf die Ausführung in der Produktionslinie beschränken, ohne daß ein zu großes Risiko der Weiterbenutzung unbrauchbarer Kunststoffbehälter in Kauf genommen werden müßte. Bei der erfindungsgemäßen Untersuchungsmethode ist es zudem nicht erforderlich, die ungebrochenen Griffleisten der Kunststoffbehälter einer mechanischen Beanspruchung auszusetzen. Die Griffleisten werden bei der einen Verfahrensalternative lediglich unter geringster Kraftanwendung so abgetastet, daß feststellbar ist, ob sich die beiden Griffleistenteile einer gebrochenen Griffleiste gegeneinander ver-

0039494
MERTENS & KEIL
PATENTANWÄLTE

schieben oder nicht, während bei der anderen Verfahrensalternative überhaupt keine Berührung erfolgen muß. Schließlich
ist das erfindungsgemäße Verfahren während des Transports
der Kunststoffbehälter in der Produktionslinie ausführbar,
ohne daß dieser unterbrochen werden müßte. Der normale Behandlungsablauf der Brauerei oder des Getränkebetriebes braucht
also nicht geändert zu werden.

Eine Vorrichtung zur Durchführung des zuvor geschilderten Verfahrens
ist vorzugsweise gekennzeichnet durch je mindestens einen einer Griffleiste von innen und/oder in räumlichem und/oder zeitlichem Abstand
von außen zuordenbare und unter voreinstellbarer Kraft an die jeweilige Griffleiste andrückbare und mit einer Positionsmeldeeinrichtung
zusammenwirkenden Abstastkopf. Mit einer solchen Vorrichtung läßt sich
die Abtastung der Kunststoffbehälter auf Bruch einer Griffleiste in der
Produktionslinie unter sehr geringer Krafteinwirkung leicht, sicher und
schnell ausführen, da sich der Kunststoffbehälter während der Abtastung in der Produktionslinie weiterbewegen kann und alle auf Bruch
abzutastenden Bereiche der Griffleiste an dem Abtastkopf entlanggeführt
abgetastet werden.

Vorzugsweise sind einer jeweiligen Griffleiste zwei Abtastköpfe in
räumlichem und/oder zeitlichem Abstand zuordenbar, von welchen
Abtastköpfen wenigstens einer an die Griffleiste von innen und wenigstens der andere an die Griffleiste von außen andrückbar ist. Auf
diese Weise können auch Schrägbrüche der Griffleiste mit Sicherheit
festgestellt werden.

Zweckmäßigerweise ist der jeweilige Abtastkopf als auf einer Außenfläche der jeweiligen Griffleiste abrollbare Rolle ausgebildet.

Die Positionsmeldeeinrichtung kann mit einer den jeweiligen Abtastköpfen
in der Produktionslinie nachgeordneten Ausschubeinrichtung so zusammenarbeiten, daß bei Feststellung des Bruchs wenigstens einer Griffleiste der entsprechende Kunststoffbehälter aus der Produktionslinie
ausgestoßen wird.

Mit der Erfindung wird auch vorgeschlagen, daß die Positionierungseinrichtung einen von dem jeweiligen Abtastkopf direkt oder indirekt betätigbaren Schalter in einem elektrischen Meldekreis oder eine von dem jeweiligen Abtastkopf direkt oder indirekt betätigbare mechanische Hebelanordnung aufweist. Insbesondere für die Überprüfung von Flaschentransportkästen von rechteckigem Querschnitt mit an allen vier Seiten vorgesehenen Griffleisten sollten zweckmäßigerweise bei einem Durchlauf durch die erfindungsgemäße Vorrichtung alle vier Griffleisten abgetastet werden. Zu diesem Zweck ist eine Anordnung zum Drehen des Kunststoffbehälters nach der Abtastung des ersten in Transportrichtung verlaufenden Paares von Griffleisten um eine vertikale Achse zur nachfolgenden Abtastung des zweiten, dann in Transportrichtung verlaufenden Paares von Griffleisten vorgesehen. Diese Drehanordnung kann entweder in derjenigen Position des Kunststoffbehälters, in welcher dieser - bei Bewegung des jeweiligen Abtastkopfes längs der zugeordneten Griffleiste kurzzeitig angehalten - sowohl von innen als auch von außen im Griffleistenbereich abgetastet wird, vorgesehen sein, oder bei kontinuierlichem Weitertransport der Kunststoffbehälter während der Abtastung zwischen zwei in Transportrichtung hintereinander angeordneten Abtastvorrichtungen für je ein Griffleistenpaar. Man kann aber auch einen Kunststoffbehälter einmal quer und einmal längs durch eine einzige Abtasteinrichtung zur Abtastung je eines Griffleistenpaares laufen lassen.

0039494

MERTENS & KEIL
PATENTANWÄLTE

Vorzugsweise führt man das Ultraschall-Prüfverfahren mit gerichteter Schrägeinschallung in die jeweilige Griffleiste aus, da auf diese Weise mit größerer Sicherheit alle Brüche und auch kleinere Risse in den Griffleisten festgestellt werden können.

Zur akustischen Ankopplung des Schallprüfkopfes an die Griffleiste kann es zweckmäßig sein, in den Führungsspalt zwischen Schallprüfkopf und Griffleiste Kontaktflüssigkeit, z.B. Wasser, einzusprühen. Auch dies ist im kontinuierlichen Verfahren einfach durchführbar.

Eine Vorrichtung zur Durchführung des zuvor erörterten erfindungsgemäßen Verfahrens ist beispielsweise gekennzeichnet durch mindestens einen benachbart der Produktionslinie je einer in Transportrichtung verlaufenden Griffleiste zugeordneten Schallprüfkopf. An diesem mindestens einen Schallprüfkopf, der im Spezialfall des Impuls-Echo-Verfahrens als Sender-Empfänger ausgebildet sein kann, wird also die Griffleiste des zu überprüfenden Kunststoffbehälters in der Produktionslinie zwangsläufig vorbeigeführt.

Vorzugsweise ist der jeweilige Schallprüfkopf an die zugeordnete Griffleiste elastisch andrückbar. Auf diese Weise können ohne wesentliche Beeinträchtigung des Prüfverfahrens Unebenheiten der Griffleiste, an welcher der Schallprüfkopf während des Weitertransportes des zu überprüfenden Kunststoffbehälters angedrückt wird, ausgeglichen werden.

Wenn der jeweilige Schallprüfkopf einen griffleistenseitigen Auflaufschuh trägt, kann sichergestellt werden, daß, unabhängig von gewissen

0039494
MERTENS & KEIL
PATENTANWÄLTE

Lagedifferenzen der abzutastenden Griffleiste der Schallprüfkopf zuverlässig an der Griffleiste entlanggeführt wird.

Dem jeweiligen Führungsspalt zwischen Griffleiste und Auflaufschuh ist dabei zweckmäßigerweise eine Flüssigkeitssprühdüse zugeordnet, mit Hilfe welcher die Kontaktflüssigkeit, z.B. Wasser, in den Führungsspalt einsprühbar ist.

Insbesondere für die Überprüfung von Flaschentransportkästen von rechteckigem Querschnitt mit an allen vier Seiten vorgesehenen Griffleisten sollten zweckmäßigerweise bei einem Durchlauf durch die erfindungsgemäße Vorrichtung alle vier Griffleisten abgetastet werden. Zu diesem Zweck ist eine Anordnung zum Drehen des Kunststoffbehälters nach der Abtastung der ersten in Transportrichtung verlaufenden Griffleiste oder des ersten Paares von Griffleisten um eine vertikale Achse zur nachfolgenden Abtastung der zweiten, dann in Transportrichtung verlaufenden Griffleiste oder des zweiten Paares von Griffleisten vorgesehen. Diese Drehanordnung kann entweder in derjenigen Position des Kunststoffbehälters, in welcher dieser - bei Bewegung des jeweiligen Schallprüfkopfes längs der zugeordneten Griffleiste kurzzeitig angehalten - im Griffbereich abgetastet wird, vorgesehen sein, oder bei kontinuierlichem Weitertransport der Kunststoffbehälter während der Abtastung zwischen je zwei in Transportrichtung hintereinander angeordneten Prüfeinrichtungen für je eine Griffleiste oder je ein Griffleistenpaar. Man kann aber auch einen Kunststoffbehälter einmal quer und einmal längs durch eine einzige Prüfeinrichtung zur Abtastung je eines Griffleistenpaares laufen lassen.

Weitere Merkmale, Vorteile und Anwendungsmöglichkeiten der vorliegenden Erfindung ergeben sich aus der nachfolgenden Beschreibung von Ausführungsbeispielen anhand der beiliegenden Zeichnung. Dabei bilden alle beschriebenen und/oder bildlich dargestellten Merkmale für sich oder in beliebiger sinnvoller Kombination den Gegenstand der vorliegenden Erfindung auch unabhängig von ihrer Zusammenfassung oder ihrer Rückbeziehung.

Es zeigt:

Fig. 1 in Seitenansicht schematisch einer die Erfindung verwirklichenden Vorrichtung an der Transporteinrichtung
einer Produktionslinie,

Fig. 2 die Vorrichtung von Fig. 1 in Draufsicht,

Fig. 3 in Seitenansicht schematisch eine andere Ausführungsform einer die Erfindung verwirklichende Vorrichtung
an der Transporteinrichtung einer Produktionslinie,

Fig. 4 die Vorrichtung von Fig. 3 in Draufsicht,

Fig. 5a gegenüber Fig. 3 vergrößert den Schallprüfkopf im
Bereich der Griffleiste bei Abtastung eines (vollständigen) Bruches der Griffleiste,

Fig. 5b das bei der Abtastung gemäß Fig. 5a entstehende Schallecho-Signal,

Fig. 6a eine Darstellung entsprechend Fig. 5a bei Abtastung
eines Risses in der Griffleiste, und

Fig. 6b das bei der Abtastung gemäß Fig. 6a sich ergebende
Schalecho-Signal.

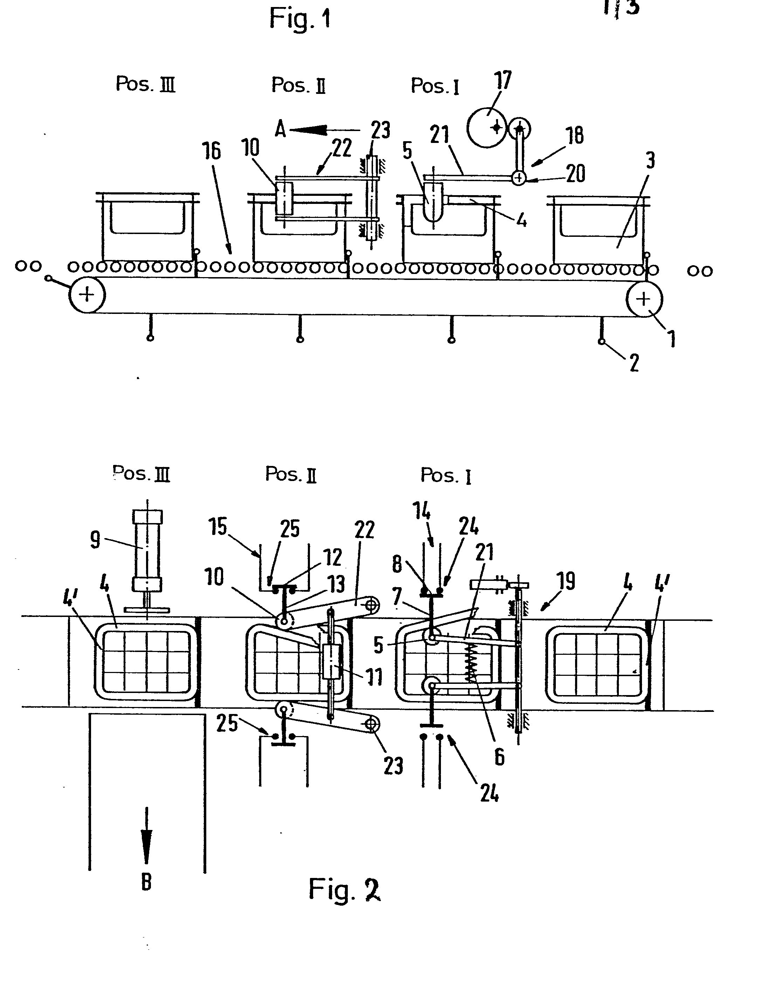

Gemäß Fig. 1 werden Kunststoffbehälter, im dargestellten Fall Flaschentransportkästen 3 mit zwei Paaren von Griffleisten 4, 4' in der Produktionslinie auf einer Rollenbahn 16 mit Hilfe einer Antriebswalzen
und ein Förderband aufweisenden Transporteinrichtung 1 in Pfeilrichtung A gefördert, indem sich an dem Transportband in Abständen
Mitnehmerleisten 2 befinden, welche am oberen Trumm die Rollenbahn 16
übergreifen. Die Flaschentransportkästen 3 kommen dabei zunächst in
die Position I. In der Position I können zwei als Rollen ausgebildete
Abtastköpfe 5 von einer Kurvenscheibe 17 aus, deren Drehung beispielsweise durch eine Lichtschranke ausgelöst wird, über ein Hebelgestänge
18, welches eine in Lagern 19 drehbare Welle 20 aufweist, in das
Innere des zu untersuchenden Flaschentransportkastens 3 so abgesenkt
werden, daß die Rollen 5 mit im wesentlichen vertikaler Drehachse
unter Weiterbewegung des Flaschentransportkastens 3 im wesentlichen
über die gesamte Innenfläche der beiden einander gegenüberliegenden
auf der Längsseite des Flaschentransportkastens 3 vorgesehenen Griffleisten 4 abrollen. Die Rollen 5 werden dabei von einem als Druckfeder
ausgebildeten, zwischen an ihrem Vorderende die Rollen 5 tragenden
Schwenkhebeln 21 angreifenden Krafterzeuger 6 mit einer geringen Kraft
nach außen gedrückt, die jedoch ausreicht, einen z.B. entsprechend der
Darstellung von Fig. 2 gebrochenen Griffteil nach außen zu biegen.
Die Rollen 5 sind am unteren Rand abgerundet. Der Krafterzeuger 6
bringt im entspannten Zustand die Rollen 5 gerade nur in einen solchen
Abstand, daß die Rollen 5 unter Berücksichtigung ihres Abstandes,
ihres Durchmessers und der unteren Abrundung gerade noch unter
Zusammenbewegung von dem Hebelgestänge 18 gegen die Kraft der
Druckfeder 6 in das Innere des Flaschentransportkastens 3 eingesetzt
werden können. An den die Rollen 5 tragenden Schwenkhebel 21 ist
jeweils ein nach außen weisender Hebel 7 als Bestandteil eines Kontaktschalters 8 angebracht. Die Kontakte 24 des Schalters 8 befinden sich
in einem solchen Abstand neben der Transporteinrichtung 1, daß bei
nicht gebrochener Griffleiste 4 (vergl. die untere Griffleiste des Flaschentransportkastens 3 in Position I von Fig. 2 ) der Kontaktteil des
Hebels 7 nicht mit den Kontakten 24 in Berührung kommt. Ist aber ein
Bruch in der Griffleiste 4 vorhanden, so kann der Hebel 7 unter
Ausbiegung des gebrochenen Teiles der Griffleiste 4 und unter Ausschwenkung des Schwenkhebels 21 mit der Rolle 5 unter der Kraft der Druckfeder 6 so weit nach außen gelangen, daß der Kontaktteil des Hebels 7 mit den zugeordneten Kontakten 24 des Schalters 8 in Berührung kommt (vgl. die obere (gebrochene) Griffleiste 4 des Flaschentransportkastens 3 in Position 1 von Fig. 2 ). Die Kontakte 24 des Schalters 8 liegen in einem elektrischen Meldekreis 14, der mit einer in Transportrichtung A nachgeordneten Ausschubeinrichtung 9 so in Verbindung steht und auf diese einwirkt, daß der Flaschentransportkasten 3, bei welchem ein Bruch der Griffleiste 4 signalisiert wurde, in Pfeilrichtung B aus der Produktionslinie seitlich ausgestoßen wird. Durch abschließende Betätigung der Kurvenscheibe 17 werden die Rollen 5 mmittels des Hebelgestänges 18 wieder aus dem untersuchten Flaschentransportkasten 3 ausgehoben.

In einer der Position I in Transportrichtung nachgeordneten Position II durchläuft der jeweilige Flaschentransportkasten 3 dann zwei von außen auf den Griffleisten 4 als abrollende Rollen ausgebildete Abtastköpfe 10. Die Rollen 10 sind um im wesentlichen vertikale Achsen drehbar am vorderen Ende von Schwenkarmen 22 gelagert, die an ihrem rückwärtigen Ende z.B. durch Befestigung an vertikal drehbar gelagerten Wellen 23 schwenkbar gehalten sind. Die Schwenkarme 22 sind über einen Krafterzeuger 11, der beispielsweise eine Zugfeder aufweisen kann, so miteinander verbunden, daß in der entspannten Stellung die Rollen 10 unter Schräglage der Schwenkarme 22 so im Bewegungsweg des jeweiligen Flaschentransportkastens 3 liegen, daß unter der Bewegung des Flaschentransportkastens 3 und der dadurch bedingten Ausschwenkung der Schwenkarme 22 die Rollen 10 an der Außenfläche der jeweiligen Griffleiste 4 abrollen können, wenn der Flaschentransportkasten 3 vorbeitransportiert wird. An den Schwenkarmen 22 sind ähnlich wie an den Schwenkhebeln 21 nach außen weisende Hebel 13 eines jeweiligen Kontaktschalters 12 angebracht, wobei die Kontakte 25 des jeweiligen Schalters 12 in einem solchen Abstand von der Produktionslinie liegen, daß beim Abrollen der jeweiligen Rolle 10 auf einer ungebrochenen Griffleiste 4 der kontaktbildende Teil des Hebels 13 nicht mit den

0039494

MERTENS & KEIL

PATENTANWÄLTE

Kontakten 25 in Berührung kommt (vergl. die untere Griffleiste 4 in Position II von Fig. 2 ). Ist aber die Griffleiste 4 gebrochen, so wird der gebrochene Teil unter der Wirkung des Krafterzeugers 11 über den jeweiligen Schwenkarm 22 und die Rolle 10 nach innen gebogen, bis der kontaktbildende Teil des Hebels 13 des Schalters 12 mit den zugeordneten Kontakten 25 in Berührung kommt (vergl. die obere Griffleiste 4 in Position II von Fig. 2 ). In diesem Fall wird wiederum ein mit den Kontakten 25 in Verbindung stehender Meldekreis 15 geschlossen, wodurch die nachgeordnete Ausschubeinrichtung 9, so zeitlich verzögert, betätigt wird, daß der beschädigte Flaschentransportkasten 3 in Richtung B seitlich aus der Produktionslinie ausgestoßen wird. Die Meldekreise 14 und 15 wirken so, daß ein seitlicher Ausstoß des Flaschentransportkastens 3 über die Ausstoßeinrichtung 9 jedenfalls dann erfolgt, wenn wenigstens einer der Schalter 8 und 12 beim Durchlauf des betreffenden Flaschentransportkastens 3 geschlossen wird. Wie man sieht, dienen also die beiden nacheinander angeordneten Rollenabtaster der Untersuchung der in Transportrichtung liegenden längsseits verlaufenden Griffleisten 4 eines Flaschentransportkastens 3 auf Brüche der beiden möglichen Schräglagen, die bei einer Abtastung nur von innen oder nur von außen nicht beide erfaßt werden könnten.

Bei der Vorrichtung gemäß Fig.1 und 2 werden die längs verlaufenden, also längeren Griffleisten 4 des jeweiligen Flaschentransportkastens 3 paarweise auf Bruch abtastend untersucht, während der jeweilige Flaschentransportkasten 3 weitertransportiert wird. Es ist jedoch auch möglich, nach entsprechender Drehung des Flaschentransportkastens 3 die beiden kürzeren Griffleisten 4' in entsprechender Weise auf Bruch abzutasten. Dabei kann beispielsweise ein Flaschentransportkasten 3 eine einzige der dargestellten Abtasteinrichtungen einmal längs und einmal quer durchlaufen, oder man schaltet zwei solcher Abtasteinrichtungen hintereinander und sieht zwischen ihnen eine geeignete Drehanordnung vor. Ferner ist es möglich, die Griffleisten 4, 4' einzeln abzutasten und auch die Abtastköpfe 5, 10 bei stillstehendem Kunststoffbehälter 3 an den Griffleisten 4, 4' entlangzubewegen.

Die Betätigung der Ausstoßeinrichtung 9 kann anstatt über ein elektrisches Meldesystem, wie dargestellt, auch durch ein Hebelsystem mechanisch erfolgen.

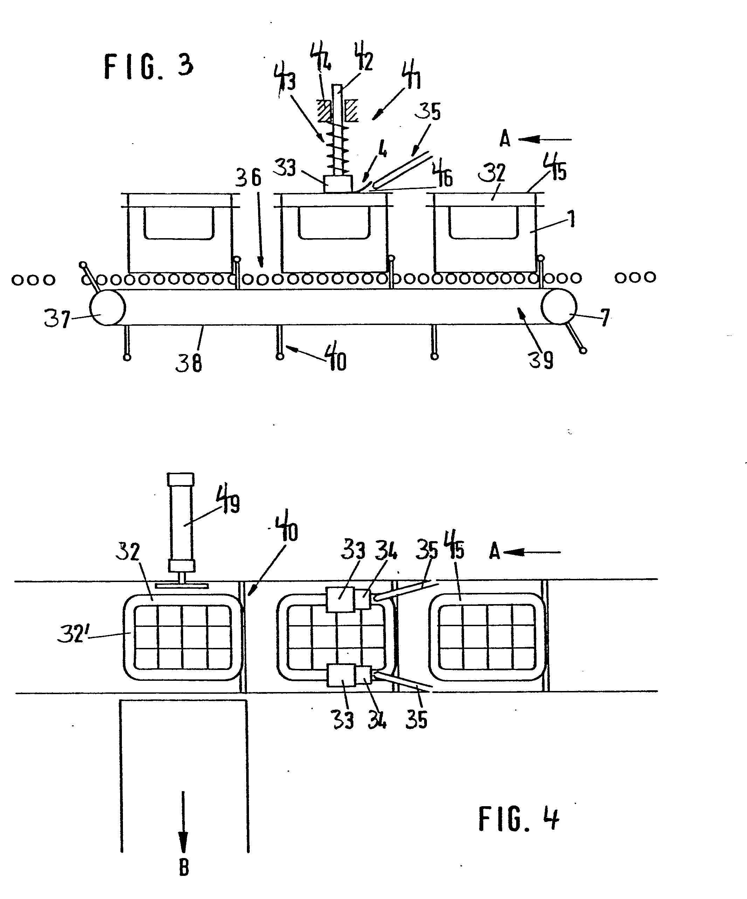

Gemäß Fig. 3 werden Kunststoffbehälter, im dargestellten Fall Flaschentransportkästen 31, mit zwei Paaren von Griffleisten 32, 32' in der Produktionslinie auf einer Rollenbahn 36 mit Hilfe einer Antriebswalzen 37 und ein Förderband 38 aufweisenden Transporteinrichtung 39 in Pfeilrichtung A gefördert, indem sich an dem Transportband 38 in Abständen Mitnehmerleisten 40 befinden, welche am oberen Trum die Rollenbahn 36 übergreifen. Die Flaschentransportkästen 31 kommen dabei in den Bereich einer oberhalb der Transporteinrichtung 39 angeordneten Prüfeinrichtung 41, die je einen einer Griffleiste 32 zugeordneten Schallprüfkopf 33 aufweist, welcher mit Hilfe einer in einem Lager 44 verschieblich gehaltenen Führungsstange 42 und einer Druckfeder 43 - im dargestellten Falle - an die Oberseitenfläche 45 der jeweiligen Griffleiste 32 andrückbar ist und an dieser entweder bei Weiterbewegung des Flaschentransportkastens 31 oder bei Bewegung des Schallprüfkopfes 33 in Richtung A oder -A entlanggleiten kann. Der jeweilige Schallprüfkopf 33 weist griffleistenseitig einen Auflaufschuh 34 auf. In den jeweiligen Führungsschlitz 46 zwischen Auflaufschuh 34 und Griffleiste 32 ist von einer Flüssigkeitssprühdüse 35 aus jeweils Kontaktflüssigkeit, z.B. Wasser, einsprühbar.

Aufgrund des Federdruckes und der Flüssigkeitseinsprühung erfolgt eine gute akustische Ankopplung des Schallprüfkopfes 33 an die Griffleiste 32 und zwar während der gesamten Zeit, während welcher sich der jeweilige Kunststoffbehälter 31 an dem Schallprüfkopf 33 (relativ) vorbeibewegt. Auf diese Weise wird die jeweilige Griffleiste 32 über ihre gesamte Länge akustisch abgetastet.

0039494
MERTENS & KEIL
PATENTANWÄLTE

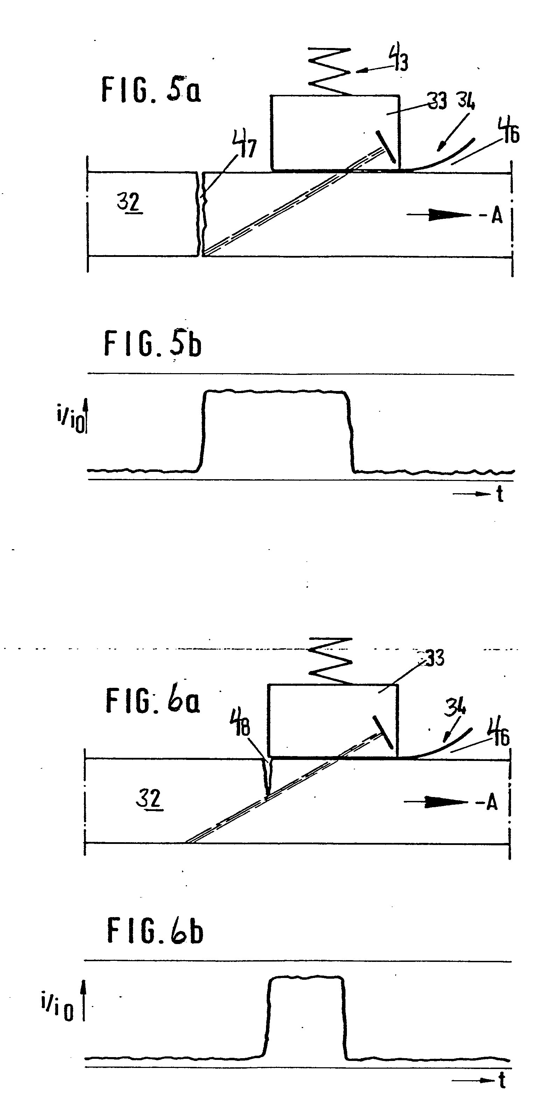

Der jeweilige Schallprüfkopf 33 arbeitet bevorzugt nach dem Impulsecho-Verfahren und zwar gemäß Fig. 5a und 6a unter gerichteter Schrägeinschallung in die Griffleiste 32. Auf diese Weise werden von dem als Sender und Empfänger ausgebildeten Schallprüfkopf 33 sowohl (vollständige) Brüche 47 (Fig. 5a), aber auch kleinere Risse 48 (Fig. 6a) feststellbar. Aus der Form, Lage und Höhe des Signals (vgl. Fig. 5b und Fig. 6b) läßt sich beispielsweise die Tiefe des Risses 48 feststellen, die als Maß für die Notwendigkeit des Ausscheidens des so beschädigten Kunststoffbehälters 31 genommen werden kann.

von dem jeweiligen Schallprüfkopf 33 wird an eine der Position der Prüfeinrichtung 41 in Transportrichtung nachgeordnete Ausschubeinrichtung 49, entsprechend zeitlich verzögert, immer dann mit einem Signal so beaufschlagt, daß ein seitliches Ausstoßen des betreffenden, aufgrund eines Bruches 47 oder hinreichend großen Risses 48 für unbrauchbar gefundenen Kunststoffbehälters 31 in Richtung B erfolgt.

Bei der dargestellten Prüfeinrichtung 41 werden die Griffleisten 32 je paarweise abgetastet und zwar jeweils das Griffleistenpaar, welches in Transportrichtung verläuft, da auf diese Weise während der Ausführung des Prüfverfahrens der Kunststoffbehälter 31 weitertransportiert wird. Zur Prüfung des zunächst quer verlaufenden Griffleistenpaares kann nach der ersten Prüfung eine Drehung des Kunststoffbehälters 31 um eine vertikale Achse in einer der Prüfeinrichtung 41 nachgeordneten Drehanordnung erfolgen, wonach der Kunststoffbehälter 31 eine weitere oder die gleiche Prüfeinrichtung 41 (nochmals) durchläuft. Natürlich ist es entsprechend auch möglich, die Griffleisten 32 je einzeln zu überprüfen.

MERTENS & KEIL
PATENTANWÄLTE

Bezugszeichenliste:

1      Transporteinrichtung

2      Mitnehmerleisten

3      Flaschentransportkästen

4, 4'    Griffleisten

5      Abtastkopf

6      Krafterzeuger

7      Hebel

8      Schalter

9      Ausschubeinrichtung

10     Abtastkopf

11     Krafterzeuger

12     Schalter

13     Hebel

14     Meldekreis

15     Meldekreis

16     Rollenbahn

17     Kurvenscheibe

18     Hebelgestänge

19     Lager

20     Welle

21     Schwenkhebel

22     Schwenkarm

23     Welle

24     Kontakte

25     Kontakte

| 31 | Kunststoffbehälter |
| 32, 32' | Griffleiste |
| 33 | Schallprüfkopf |
| 34 | Auflaufschuh |
| 35 | Flüssigkeitssprühdüsen |
| 36 | Rollenbahn |
| 37 | Antriebswalzen |
| 38 | Förderband |
| 39 | Transporteinrichtung |
| 40 | Mitnehmerleisten |
| 41 | Prüfeinrichtung |
| 42 | Führungsstange |
| 43 | Feder |
| 44 | Lager |
| 45 | Oberseitenfläche |
| 46 | Führungsspalt |
| 47 | Brüche |
| 48 | Risse |
| 49 | Ausschubeinrichtung |

0039494

**MERTENS & KEIL**

PATENTANWÄLTE

Frankfurt am Main

30.4.1981

T 12 P 13/14 EP

K/De

Heinz Till

Fischbacher Weg 28

6238 Hofheim


Volker Till

Eichendorffstr. 17

6238 Hofheim

"Verfahren und Vorrichtung zur Überprüfung und Gewährleistung
der Brauchbarkeit von Kunststoffbehältern mit Griffleisten,
z.B. Flaschentransportkästen"


Ansprüche:


1. Verfahren zur Überprüfung und Gewährleistung der Brauchbarkeit von Kunststoffbehältern mit Griffleisten, z.B. Flaschentransportkästen aus Polyäthylen oder dergl., dadurch gekennzeichnet, daß man die Kunststoffbehälter (3) innerhalb einer
Produktionslinie im Bereich der Griffleisten (4) auf Brüche
oder Risse selbsttätig mechanisch oder mit Hilfe eines Ultraschall-Prüfverfahrens, z.B. des Ultraschall-Durchschallung-
und/oder Impulsecho-Verfahrens abtastet und bei Feststellung
eines Bruches aus der Produktionslinie ausstößt.

0039494

MERTENS & KEIL
PATENTANWÄLTE

2. Vorrichtung zur Durchführung des Verfahrens nach Anspruch 1, gekennzeichnet durch je mindestens eine einer Griffleiste (4) von innen und in räumlichem und/oder zeitlichem Abstand von außen zuordenbare und unter voreinstellbarer Kraft an die jeweilige Griffleiste (4) andrückbare und mit einer Positionsmeldeeinrichtung zusammenwirkenden Abtastkopf (5, 10).

3. Vorrichtung nach Anspruch 2, dadurch gekennzeichnet, daß der jeweiligen Griffleiste (4) zwei Abtastköpfe (5, 10) in räumlichem und/oder zeitlichem Abstand zuordenbar sind, von welchen wenigstens der eine an die Griffleiste (4) von innen und wenigstens die andere an die Griffleiste (4) von außen andrückbar ist.

4. Vorrichtung nach Anspruch 2 oder 3, dadurch gekennzeichnet, daß der jeweilige Abtastkopf (5, 10) als Rolle ausgebildet ist.

5. Vorrichtung nach einem der Ansprüche 2 bis 4, dadurch gekennzeichnet, daß die Positionsmeldeeinirichtung mit einer den jeweiligen Abtastköpfen (5, 10) in der Produktionslinie nachgeordneten Ausschubeinrichtung (9) zusammenarbeitet.

6. Vorrichtung nach einem der Ansprüche 2 bis 5, dadurch gekennzeichnet, daß die Positionsmeldeeinrichtung einen von dem jeweiligen Abtastkopf (5, 10) direkt oder indirekt betätigbaren Schalter (8, 12) in einem elektrischen Meldekreis (14, 15) oder eine von dem jeweiligen Abtastkopf (5, 10) direkt oder indirekt betätigbare mechanische Hebelanordnung aufweist.

0039494

MERTENS & KEIL
PATENTANWÄLTE

7. Verfahren nach Anspruch 1, dadurch gekennzeichnet, daß man das Ultraschall-Prüfverfahren mit gerichteter Schrägeinschallung in die jeweilige Griffleiste (32) ausführt.

8. Verfahren nach Anspruch 1 oderr 7, dadurch gekennzeichnet, daß man zur akustischen Ankopplung in den Führungsspalt (46) zwischen Schallprüfkopf (33) und Griffleiste (32) Kontaktflüssigkeit, z.B. Wasser, einsprüht.

9. Vorrichtung zur Durchführung des Verfahrens nach einem der Ansprüche 1, 7 oder 8, gekennzeichnet durch mindestens einen benachbart der Produktionslinie je einer in Transportrichtung verlaufenden Griffleiste (32) zugeordneten Schallprüfkopf (33).

10. Vorrichtung nach Anspruch 9, dadurch gekennzeichnet, daß der jeweilige Schallprüfkopf (33) an die zugeordnete Griffleiste (32) elastisch andrückbar ist.

11. Vorrichtung nach Anspruch 9 oder 10, dadurch gekennzeichnet, daß der jeweilige Schallprüfkopf (33) einen griffleistenseitigen Auflaufschuh (34) trägt.

12. Vorrichtung nach einem der Ansprüche 9 bis 11, dadurch gekennzeichnet, daß dem jeweiligen Führungsspalt (46) zwischen Griffleiste (32) und Auflaufschuh (34) eine Flüssigkeitssprühdüse (35) zugeordnet ist.

13. Vorrichtung nach einem der Ansprüche 9 bis 12, insbesondere für die Abtastung der Griffleisten von Flaschentransportkästen, gekennzeichnet durch eine Anordnung zum Drehen des Kunststoffbehälters (31) nach der Abtastung der ersten, in Transportrichtung verlaufenden Griffleiste (32) oder des ersten Paares von Griffleisten (32) um eine vertikale Achse zur nachfolgenden Abtastung der zweiten, dann in Transportrichtung verlaufenden Griffleiste (32) oder des zweiten Paares von Griffleisten (32).

**Fig. 1**

Pos. III    Pos. II    Pos. I

**Fig. 2**

Pos. III    Pos. II    Pos. I

FIG. 3

43  44  42  41  35  A

33  4  46  32  45

36

33

37  1

38  40  39  7

49

32  40  33 34 35 45  A

32

32'

33 34 35

33 34 35

B

FIG. 4

FIG. 5a

FIG. 5b

FIG. 6a

FIG. 6b

$i/i_0$

$i/i_0$

A

A

t

t

32

32

43

33 34

46

47

33

48

34

46

Europäisches
Patentamt

**EUROPÄISCHER RECHERCHENBERICHT**

0039494
Nummer der Anmeldung

EP 81 10 3293.7

| Kategorie | Kennzeichnung des Dokuments mit Angabe, soweit erforderlich, der maßgeblichen Teile | betrifft Anspruch | KLASSIFIKATION DER ANMELDUNG (Int. Cl.³) |
|---|---|---|---|
| | **EINSCHLÄGIGE DOKUMENTE** | | |
| | DE - B1 - 2 922 535 (BEROLINA KUNST-STOFF - GMBH) <br> * Spalte 4, Zeilen 6 bis 25 * <br> -- | 1,2,5, 6,13 | G 01 M   19/00 |
| A | US - A - 3 745 833 (D.A. ARMSTRONG) <br> * Anspruch 1 * <br> -- | 8,12 | |
| A | DE - A - 1 573 614 (J.u.H. KRAUTKRÄMER GESELLSCHAFT F. ELEKTROPHYSIK) <br> * Seite 1, Zeilen 6 bis 8 * <br> ---- | 7 | |

**RECHERCHIERTE SACHGEBIETE (Int. Cl.³)**

G 01 M   19/00
G 01 N   29/04

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patent-familie, übereinstimmendes Dokument

☒ Der vorliegende Recherchenbericht wurde für alle Patentansprüche erstellt.

| Recherchenort | Abschlußdatum der Recherche | Prüfer |
|---|---|---|
| Berlin | 12-08-1981 | BOTTERILL |

EPA form 1503.1  06.78